# EUROPEAN PATENT SPECIFICATION

(11) **EP 4 246 136 B1**
(45) Date of publication and mention of the grant of the patent: **09.12.2026**
(21) Application number: 21890918.2
(22) Date of filing: 21.10.2021
(51) Int. Cl.: G01N 25/20, G01N 17/00, G01N 25/02, G01N 33/44

(54) **METHOD FOR DETECTING SERVICE LIFE OF PRE-CROSSLINKED MATERIAL FOR HIGH-VOLTAGE ALTERNATING-CURRENT CABLE INSULATION**
VERFAHREN ZUR ERKENNUNG DER LEBENSDAUER VON VORVERNETZTEM MATERIAL ZUR HOCHSPANNUNGSWECHSELSTROMKABELISOLIERUNG
PROCÉDÉ DE DÉTECTION DE DURÉE DE VIE D'UN MATÉRIAU PRÉ-RÉTICULÉ D'ISOLATION DE CÂBLE À COURANT ALTERNATIF HAUTE TENSION

(30) Priority: 11.11.2020 CN 202011250164
(43) Date of publication of application: 20.09.2023
(73) Proprietor: Electric Power Research Institute, China Southern Power Grid, Guangzhou, Guangdong 510663 (CN); Shenzhen Power Supply Co., Ltd., Shenzhen, Guangdong 518001 (CN)
(72) Inventor: LI, Xiaolin, Guangzhou, Guangdong 510663 (CN); FU, Mingli, Guangzhou, Guangdong 510663 (CN); HOU, Shuai, Guangzhou, Guangdong 510663 (CN); HUI, Baojun, Guangzhou, Guangdong 510663 (CN); ZHU, Wenbo, Guangzhou, Guangdong 510663 (CN); WU, Guoxing, Guangzhou, Guangdong 510663 (CN); XIE, Hong, Guangzhou, Guangdong 510663 (CN); CHEN, Xiao, Guangzhou, Guangdong 510663 (CN); XU, Shu, Guangzhou, Guangdong 510663 (CN); FENG, Bin, Guangzhou, Guangdong 510663 (CN); ZHANG, Yifan, Guangzhou, Guangdong 510663 (CN)
(74) Representative: Hoffmann Eitle
(86) International application number: PCT/CN2021/125231
(87) International publication number: WO 2022/100391

(56) References cited:
- WO-A1-2014/197550
- CN-A- 104 793 111
- CN-A- 105 158 085
- CN-A- 105 866 015
- CN-A- 108 828 416
- CN-A- 109 917 251
- CN-A- 110 186 513
- CN-A- 111 337 418
- CN-A- 111 366 459
- CN-A- 112 557 438
- CN-B- 101 531 783
- CHEN XIANGRONG ET AL: "Effect of thermal ageing on charge dynamics and material properties of 320 kV HVDC XLPE", IEEE TRANSACTIONS ON DIELECTRICS AND ELECTRICAL INSULATION, IEEE SERVICE CENTER, PISCATAWAY, NJ, US, vol. 26, no. 6, 1 December 2019 (2019-12-01), pages 1797 - 1804, XP011753817, ISSN: 1070-9878, [retrieved on 20191204], DOI: 10.1109/TDEI.2019.008160
- ZHANG YUANYUAN ET AL: "Non-debye dielectric behavior of aged XLPE cable insulation", 2018 12TH INTERNATIONAL CONFERENCE ON THE PROPERTIES AND APPLICATIONS OF DIELECTRIC MATERIALS (ICPADM), IEEE, 20 May 2018 (2018-05-20), pages 435 - 438, XP033368767, DOI: 10.1109/ICPADM.2018.8401040

## Description

### TECHNICAL FIELD

This disclosure relates to the field of performance testing of high-voltage alternating-current cable insulation materials, in particular to a method for detecting storage life of pre-crosslinked material for high-voltage alternating-current cable insulation.

### BACKGROUND

The insulation layer of high-voltage alternating-current cable is made of crosslinked polyethylene as the main insulating material. The chemical cross-linked method is most widely used to achieve the cross-linked polyethylene. The principle is cross-linking the polyethylene with linear structure into crosslinked polyethylene with network structure by means of free radicals generated by thermal decomposition of peroxide cross-linking agents, in which the extruded crosslinked material is a pre-crosslinked material of crosslinked polyethylene. The process for producing the pre-crosslinked material includes adding an antioxidant with a mass fraction of 0.2% to polyethylene at a mixing temperature of 130-250°C, uniformly mixing in a screw mixer and adding a cross-linking agent (usually Dicumyl Peroxide, DCP) with a mass fraction of 2% at a mixing temperature of 110-120°C, uniformly mixing and extruding through a screw extruder, pelletizing, and dehydrating by passing through a vibrating screen, so as to form the finished product of pre-crosslinked material.

The cross-linking agent DCP is a strong oxidant. Before the pre-crosslinked material is used to manufacture the cable, a certain amount of DCP would be decomposed spontaneously during the storage process, resulting in a decrease of the DCP content in the pre-crosslinked material. In the formal cross-linking extrusion of the pre-crosslinked material, DCP as an initiator of cross-linking reaction, has a significant impact on the efficiency of the cross-linking reaction and the cross-linking density of the final product, so pre-crosslinked material with different storage durations will has different activities due to different DCP contents, thereby directly affecting the performance of the finished product of cable insulation layer.

Therefore, the activity and storage life of the pre-crosslinked material are crucial to the performance of the cable insulation layer. However, neither a mature method for detecting the activity and storage life of pre-crosslinked material for high-voltage alternating-current cable insulation nor a method for determining the aging time and temperature required for accelerated aging test on pre-crosslinked material does exist.

Chen X, Dai C, Yu L, et al. disclose in "Effect of thermal ageing on charge dynamics and material properties of 320 kV HVDC XLPE" (in: IEEE Transactions on Dielectrics and Electrical Insulation, 2019, 26, Nr. 6, pp. 1797-1804) reports on the effect of thermal ageing on the space charge characteristics, DC conductivities and trap energy distributions of 320 kV HVDC XLPE. Specimens were prepared by a heating press method in the laboratory. The specimens were placed in a thermal oven and thermally aged at 130 °C up to 30 days. Space charges were measured by a pulsed electro-acoustic setup. The results showed that the reference samples had limited hetero charge accumulations near both electrodes in the samples. The homo-charge accumulation was appeared with an increase of the aged time. The space charge accumulation in the samples was transferred from hetero-charge accumulation to homo-charge accumulation with an increase of the ageing time. The electric field distribution in the sample gradually increased with the appearance of the homo-charge accumulation, whereas the homo charges evidently distorted the electric field in the samples with the increasing ageing time. The DC conductivities of the aged samples consistently increased with the increase of the measured electric field. The obtained results of apparent mobility of the charge carriers, the activation energy and the trap energy density illustrated that thermal ageing could facilitate the homo-charge accumulation in the sample. The dominant effect of thermal ageing on the sample was elucidated by using various analytical techniques. It was found that the thermal ageing would affect the material properties and facilitate the increase of the trap energy density and the space charge accumulations in the samples.

CN 101531783 B discloses a delayed cross-linked type silicane cross-linked polyethylene plastic. The plastic is characterized in that a specific modification auxiliary agent is used for improving the influence extent of the plastic composition by the water-contained environment, improving the storage stability of the plastic and slowing down the pre-cross-link. The use of the modifying agent aims at generating a layer of hydrophobic groups on the material surface so as to delay the contact of polymers and water molecules and delay the cross-link.

Zhang, Yuanyuan, et al. disclose in "Non-debye dielectric behavior of aged XLPE cable insulation." (in: Proc. 2018 12th International Conference on the Properties and Applications of Dielectric Materials (ICPADM). IEEE, 2018), reports on dielectric responses of aged XLPE cable insulation. Thermal aging experiment was performed on XLPE sheets cut from 110 kV power cable insulation under 130°C for 10, 20, and 50 days, respectively. The dielectric spectra of all the samples were measured at different temperatures in a frequency range from 10⁻¹ to 10⁶ Hz. An improved analytical methodology, the derivation of real permittivity respect to natural logarithm of angular frequency (∂έ/∂lnω) was applied in this paper.

### SUMMARY

Embodiments of the present disclosure provide a method for detecting storage life of pre-crosslinked material for high-voltage alternating-current cable insulation, which can determine not only the aging time and temperature required for accelerated aging test on pre-crosslinked material but also the storage life of pre-crosslinked material for high-voltage alternating-current cable insulation.

The present invention provides for a method for detecting storage life of pre-crosslinked material for high-voltage alternating-current cable insulation according to claim 1.

Further, dielectric properties of the reference crosslinked polyethylene are measured to obtain reference data of dielectric property. Dielectric properties of the crosslinked polyethylene prepared from the expired pre-crosslinked material are measured to obtain detection data of dielectric property. The reference data of dielectric property and the detection data of dielectric property are compared to verify deterioration of the expired pre-crosslinked material.

In which, the cross-linking degree of the reference crosslinked polyethylene and the cross-linking degree of the crosslinked polyethylene prepared from the pre-crosslinked material that undergoes accelerated aging are measured by differential scanning calorimetry (DSC) test and thermal extension test.

Further, the mechanical properties include tensile strength, elastic modulus and elongation at break. The dielectric properties include direct-current conductivity, alternating-current breakdown field strength, relative dielectric constant and dielectric loss tangent.

Compared with the relative art, the present disclosure has the following beneficial effects. Embodiments of the present disclosure provide a method for detecting storage life of pre-crosslinked material for high-voltage alternating-current cable insulation, which includes taking a pre-crosslinked material and performing tabletting to obtain a crosslinked polyethylene, measuring the cross-linking degree, mechanical properties and dielectric properties of the crosslinked polyethylene to serve as reference data; subjecting the pre-crosslinked material to accelerated aging via heating and measuring the cross-linking degree, mechanical properties and dielectric properties of the crosslinked polyethylene prepared by tabletting on the pre-crosslinked material that undergoes accelerated aging, comparing the detection data with the reference data; prolonging a preset heating duration according to a preset step length in the case where the comparison result falls within an error allowable range, until the comparison result does not completely fall within the error allowable range; converting the time duration after subtracting the step length from the heating duration into a time duration under a normal temperature according to a formula for calculating a decomposition rate of a cross-linking agent and a formula for calculating the half-life as a function of temperature, so as to obtain the storage life of the pre-crosslinked material. In the present disclosure, accelerated aging is adopted via heating, thereby shortening test cycle and reducing material consumption. According to measuring the cross-linking degree and mechanical properties of the crosslinked polyethylene prepared from the pre-crosslinked material that undergoes accelerated aging, comparing the measurement values with the cross-linking degree and mechanical properties of the reference crosslinked polyethylene, and combining the decomposition rate of a cross-linking agent in the pre-crosslinked material and the change of the half-life with temperature, the aging time and temperature required for accelerated aging test on pre-crosslinked material are determined, thereby realizing the detection of the storage life of the pre-crosslinked material.

### BRIEF DESCRIPTION OF THE DRAWINGS

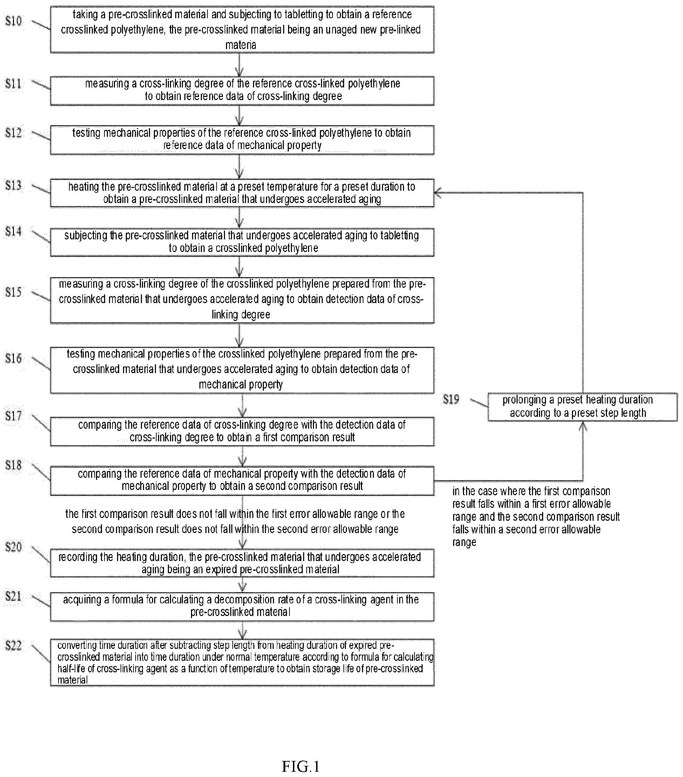

FIG. 1 is a schematic flowchart of a method for detecting storage life of pre-crosslinked material for high-voltage alternating-current cable insulation provided in an embodiment of the present disclosure.

### DETAILED DESCRIPTION

The technical solutions in the embodiments of the present disclosure will be clearly and completely described below. Obviously, the described embodiments are only a part of but not all of the embodiments of the present disclosure.

In order to illustrate the determination of parameters of the accelerated aging test and a method for calculating the same, the theory of calculating the half-life and decomposition rate of the cross-linking agent DCP is briefly introduced herein.

Studies have shown that the crosslinking of polyethylene is a first-order reaction, with a reaction kinetic equation of $v = - \frac{dC}{dt} = K ⋅ C$ (1) in formula (1), v is the reaction rate, C is the DCP concentration, and K is the reaction rate constant.

The following formula is solved from formula (1) $C = {C}_{0} ⋅ {e}^{- K ⋅ t}$ (2) in formula (2), C₀ is the initial concentration of DCP in the pre-crosslinking material.

Therefore, the following formula for calculating the decomposition rate X of DCP in the pre-crosslinked material at time t can be obtained through the reaction kinetic theory. $X = \frac{{C}_{0} - C}{{C}_{0}} = 1 - {e}^{- Kt}$ (3)

The formula (3) shows that the decomposition rate of DCP increases with the prolongation of storage duration, thereby affecting the activity of the pre-crosslinked material. The formula for calculating the reaction rate constant K can be obtained from the Arrhenius formula. $K = A ⋅ {e}^{- \frac{E}{RT}}$ (4)

In formula (4), R is the gas constant, E is the apparent activation energy, and A is the frequency factor. Therefore, at different temperatures, the ratio of the reaction rate constants is: $\frac{{K}_{2}}{{K}_{1}} = {e}^{\frac{E}{R} ⋅ \left(\frac{1}{{T}_{1}}-\frac{1}{{T}_{2}}\right)}$ (5)

The decomposition half-life of DCP τ is defined as the time required where the DCP concentration drops to the half of the initial concentration at a certain ambient temperature. The following formula can be solved by substituting τ into the formula for calculating the decomposition rate of DCP. $τ = \frac{ln 2}{K}$ (6)

The following formula can be obtained by substituting the formula (6) into the formula (3) for calculating the decomposition rate. $X = 1 - {e}^{- \frac{t ⋅ ln 2}{τ}}$ (7)

Meanwhile, the half-life of DCP in the pre-crosslinked material at another temperature is calculated according to the formula (6) indicating the relationship between the decomposition half-life of DCP τ and the reaction rate constant K, and the formula (5) for calculating the ratio of reaction rate constants, in combination with the measured half-life of DCP at a certain temperature. The calculation formula is as follows. $τ = {τ}_{0} ⋅ {e}^{\frac{E}{R} ⋅ \left(\frac{1}{T}-\frac{1}{{T}_{0}}\right)}$ (8)

In formula (8), τ₀ is the half-life of DCP at a known temperature T₀.

According to the existing experimental data and conclusions, the decomposition half-life of DCP at room temperature of 30°C is calculated to be 490 years, and the half-life at other temperatures can also be calculated. The complete calculation results are shown in Table 1 below.

**Table 1 Decomposition half-life of DCP in pre-crosslinked materials at different temperatures**

| temperature/°C | half-life | temperature/°C | half-life | temperature/°C | half-life |
|---|---|---|---|---|---|
| 30 | 490 years | 100 | 51.6 hours | 170 | 1.32 minutes |
| 40 | 72 years | 110 | 14.3 hours | 180 | 31.8 seconds |
| 50 | 12 years | 120 | 4.2 hours | 190 | 13.3 seconds |
| 60 | 784 days | 130 | 80 minutes | 200 | 5.75 seconds |
| 70 | 157 days | 140 | 26.6 minutes | 210 | 2.58 seconds |
| 80 | 35 days | 150 | 9.3 minutes | 220 | 1.19 seconds |
| 90 | 8.3 days | 160 | 3.4 minutes | 230 | 0.571 seconds |

In combination of the data in Table 1 and the calculation formula (7) of the decomposition rate of DCP, the DCP content in the pre-crosslinked material after storage at room temperature for a certain period of time can be calculated. In order to achieve the same decomposition rate of DCP in the accelerated aging test on the pre-crosslinked material, and to achieve the effect of corresponding storage period of time at equivalent room temperature, the stability of the mixture system of pre-crosslinked material and the rationality of time duration in the aging test should also be considered.

It should be noted herein that the spontaneous decomposition of DCP can indeed be accelerated by increasing the storage temperature, so as to achieve the purpose of accelerating the aging of pre-crosslinked materials. However, the polyethylene molecules in the pre-crosslinked material will also be affected, so that the polyethylene molecules will be weakly cross-linked spontaneously due to the increased number of active free radicals in the pre-crosslinked material mixing system, which will affect the subsequent test result. However, the polyethylene crosslinking phenomenon induced during the aging process can be ignored because the set temperature in the accelerated aging test is usually lower than the actual temperature of about 180°C in the crosslinking process. Moreover, from the perspective of chemical reaction principles, since the crosslinking reaction is an exothermic reaction, the increased temperature will make the reaction balance to shift to the left, and thus the crosslinking reaction will be inhibited to a certain extent and the proportion of crosslinked products in the pre-crosslinked material system is also reduced. It can be considered that the influence of the accelerated aging test on the activity of the pre-crosslinked material is mainly due to the spontaneous decomposition of DCP rather than the polyethylene molecule itself.

Based on the above conclusions, 70°C is chosen as the test temperature for the accelerated aging test. At this temperature, it can be seen from Table 1 that the decomposition half-life of DCP is 157 days. According to the calculation of formula (7), it can be seen that 3.8 hours of accelerated aging can equivalently achieve the effect of storing at 30°C for half a year, while aging for 77 hours (about 3 days) can equivalently achieve the effect of storing at room temperature for 10 years. Also, 70°C is much lower than the temperature of the crosslinking process, so this aging temperature takes into account the stability of the pre-crosslinking material system and the rationality of time duration in the aging test.

Referring to FIG. 1, FIG. 1 is a schematic flowchart of the method for detecting storage life of pre-crosslinked material for high-voltage alternating-current cable insulation provided in an embodiment of the present disclosure. The method includes the follows.

S10 A pre-crosslinked material is taken, and the pre-crosslinked material is subjected to tabletting to obtain a reference crosslinked polyethylene. The pre-crosslinked material is an unaged new pre-crosslinked material.

S11 A cross-linking degree of the reference crosslinked polyethylene is measured to obtain reference data of cross-linking degree.

S12 Mechanical properties of the reference crosslinked polyethylene are tested to obtain reference data of mechanical property.

Exemplarily, steps S10 to S12 may be specifically conducted as follows. The new pre-crosslinked material from the factory was crosslinked at 180°C, followed by mold into thin slices with a thickness of 0.2 mm and 1.0 mm respectively by using a flat vulcanizer and cut into round or dumbbell-shaped specimens by using a cutter. Among them, the round sample with a thickness of 0.2 mm is used for the measurement of dielectric properties, and the dumbbell-shaped sample with a thickness of 1.0 mm is used for the measurement of thermal extension and mechanical properties. The crosslinking degree of crosslinked polyethylene sample prepared from the new pre-crosslinked material from the factory was measured according to the DSC test in GB/T 36965-2018. The measurement value is used as the standard for subsequent evaluation of the activity and lifetime of the pre-crosslinked material.

S13 The pre-crosslinked material is heated at a preset temperature for a preset duration to obtain a pre-crosslinked material that undergoes accelerated aging.

S14 The pre-crosslinked material that undergoes accelerated aging is subjected to tabletting to obtain a crosslinked polyethylene.

S15 A cross-linking degree of the crosslinked polyethylene prepared from the pre-crosslinked material that undergoes accelerated aging is measured to obtain detection data of cross-linking degree.

S16 Mechanical properties of the crosslinked polyethylene prepared from the pre-crosslinked material that undergoes accelerated aging is tested to obtain detection data of mechanical property.

Among them, the cross-linking degree is measured by differential scanning calorimetry (DSC) test and thermal extension test. The mechanical properties include tensile strength, elastic modulus and elongation at break. The dielectric properties include direct-current conductivity, alternating-current breakdown field strength, relative dielectric constant and dielectric loss tangent.

Exemplarily, steps S13 to S16 may be specifically conducted as follows. The new pre-crosslinked material from the factory was subjected to the accelerated aging test. The new pre-crosslinked material from the factory was placed in a beaker. The beaker was placed in an oven at a constant temperature of 70°C and subjected to accelerated aging via heating for 3.8 hours, which is equivalent to storing at 30°C for half a year. After the accelerated aging, the aged pre-crosslinked material was obtained and kept standing at room temperature for 24 hours, followed by compression into a tablet, and detection of cross-linking degree and mechanical properties.

S17 The reference data of cross-linking degree is compared with the detection data of cross-linking degree to obtain a first comparison result.

S18 The reference data of mechanical property is compared with the detection data of mechanical property to obtain a second comparison result.

S19 A preset heating duration is prolonged according to a preset step length in the case where the first comparison result falls within a first error allowable range and the second comparison result falls within a second error allowable range. The cross-linking degree and mechanical properties of the crosslinked polyethylene are re-tested to obtain the detection data of cross-linking degree and the detection data of mechanical property. The detection data of cross-linking degree is compared with the reference data of cross-linking degree and the detection data of mechanical property is compared with the reference data of mechanical property until the first comparison result does not fall within the first error allowable range or the second comparison result does not fall within the second error allowable range.

S20 The heating duration is recorded in the case where the first comparison result does not fall within the first error allowable range or the second comparison result does not fall within the second error allowable range. The pre-crosslinked material that undergoes accelerated aging is an expired pre-crosslinked material.

Exemplarily, steps S19 to S20 may be specifically conducted as follows. If the aged pre-crosslinked material and specimens thereof have performance measurement results which are substantially unchanged relative to those of the new pre-crosslinked material and specimens thereof, it indicates that the pre-crosslinked material still maintains good activity after being stored at room temperature for half a year and the storage life of the pre-crosslinked material under the room temperature is more than half a year.

In order to obtain the storage life of the pre-crosslinked material at room temperature, the step length is set to heat the pre-crosslinked material at 70°C for 3.8 hours, which is equivalent to storage of half a year at 30°C. Through gradually extending the aging time in the accelerated aging test, an aged pre-crosslinked material with a longer storage duration at equivalent room temperature is obtained. After completing the aging test with extended aging time, the corresponding material is subjected to the tableting and the obtained specimens are subjected to detection of crosslinking degree and mechanical properties, until specimens having obvious changes in performance are obtained.

Further, the present method includes measuring dielectric properties of the reference crosslinked polyethylene to obtain reference data of dielectric property; measuring dielectric properties of the crosslinked polyethylene prepared from the expired pre-crosslinked material to obtain detection data of dielectric property; and comparing the reference data of dielectric property and the detection data of dielectric property to verify deterioration of the expired pre-crosslinked material.

S21 A formula for calculating a decomposition rate of a cross-linking agent in the pre-crosslinked material is acquired. $X = 1 - {e}^{- \frac{t ⋅ ln 2}{τ}}$

In the formula, τ is a half-life of the cross-linking agent at temperature T and t is a time duration that the cross-linking agent is stored in temperature T.

S22 The time duration after subtracting the step length from the heating duration of the expired pre-crosslinked material is converted into a time duration under a normal temperature according to a formula for calculating the half-life of the cross-linking agent as a function of temperature, so as to obtain the storage life of the pre-crosslinked material.

Exemplarily, step S22 may be specifically conducted as follows. The step length of the extended aging time is subtracted from the accelerated aging time of the specimens which have obvious changes in cross-linking degree and mechanical properties, the storage duration at 70°C is converted into a storage duration at 30°C, so as to obtain a storage life of the pre-crosslinked material at 30°C.

Embodiments of the present disclosure provide a method for detecting storage life of pre-crosslinked material for high-voltage alternating-current cable insulation, which includes taking a pre-crosslinked material and performing tabletting to obtain a crosslinked polyethylene, measuring the cross-linking degree, mechanical properties and dielectric properties of the crosslinked polyethylene to serve as reference data; subjecting the pre-crosslinked material to accelerated aging via heating and measuring the cross-linking degree, mechanical properties and dielectric properties of the crosslinked polyethylene prepared by tabletting on the pre-crosslinked material that undergoes accelerated aging, comparing the detection data with the reference data; prolonging a preset heating duration according to a preset step length in the case where the comparison result falls within an error allowable range, until the comparison result does not completely fall within the error allowable range; converting the time duration after subtracting the step length from the heating duration into a time duration under a normal temperature according to a formula for calculating a decomposition rate of a cross-linking agent and a formula for calculating the half-life as a function of temperature, so as to obtain the storage life of the pre-crosslinked material. In the present disclosure, accelerated aging is adopted via heating, thereby shortening test cycle and reducing material consumption. According to measuring the cross-linking degree and mechanical properties of the crosslinked polyethylene prepared from the pre-crosslinked material that undergoes accelerated aging, comparing the measurement values with the cross-linking degree and mechanical properties of the reference crosslinked polyethylene, and combining the decomposition rate of a cross-linking agent in the pre-crosslinked material and the change of the half-life with temperature, the aging time and temperature required for accelerated aging test on pre-crosslinked material are determined, thereby realizing the detection of the storage life of the pre-crosslinked material.

## Claims

1. A method for detecting storage life of pre-crosslinked material for high-voltage alternating-current cable insulation, comprising:
taking a pre-crosslinked material, and subjecting the pre-crosslinked material to tabletting to obtain a reference crosslinked polyethylene, the pre-crosslinked material being an unaged new pre-linked material,
measuring a cross-linking degree of the reference crosslinked polyethylene to obtain reference data of cross-linking degree,
testing mechanical properties of the reference crosslinked polyethylene to obtain reference data of mechanical property,
heating the pre-crosslinked material at a preset temperature for a preset duration to obtain a pre-crosslinked material that undergoes accelerated aging,
subjecting the pre-crosslinked material that undergoes accelerated aging to tabletting to obtain a crosslinked polyethylene,
measuring a cross-linking degree of the crosslinked polyethylene prepared from the pre-crosslinked material that undergoes accelerated aging to obtain detection data of cross-linking degree,
testing mechanical properties of the crosslinked polyethylene prepared from the pre-crosslinked material that undergoes accelerated aging to obtain detection data of mechanical property,
comparing the reference data of cross-linking degree with the detection data of cross-linking degree to obtain a first comparison result,
comparing the reference data of mechanical property with the detection data of mechanical property to obtain a second comparison result,
prolonging a preset heating duration according to a preset step length in the case where the first comparison result falls within a first error allowable range and the second comparison result falls within a second error allowable range, re-testing the crosslinked polyethylene to obtain the detection data of cross-linking degree and the detection data of mechanical property, and comparing with the reference data of cross-linking degree and the reference data of mechanical property until the first comparison result does not fall within the first error allowable range or the second comparison result does not fall within the second error allowable range, recording the heating duration in the case where the first comparison result does not fall within the first error allowable range or the second comparison result does not fall within the second error allowable range, the pre-crosslinked material that undergoes accelerated aging being an expired pre-crosslinked material,
acquiring a formula for calculating a decomposition rate of a cross-linking agent in the pre-crosslinked material: $X = 1 - {e}^{- \frac{t ⋅ ln 2}{τ}}$
wherein τ is a half-life of the cross-linking agent at temperature T, and t is a time duration that the cross-linking agent is stored in temperature T, and
converting a time duration after subtracting the step length from the heating duration of the expired pre-crosslinked material into a time duration under a normal temperature according to a formula for calculating the half-life of the cross-linking agent as a function of temperature, so as to obtain the storage life of the pre-crosslinked material.

2. The method for detecting storage life of pre-crosslinked material for high-voltage alternating-current cable insulation according to claim 1, further comprising:
measuring dielectric properties of the reference crosslinked polyethylene to obtain reference data of dielectric property,
measuring dielectric properties of the crosslinked polyethylene prepared from the expired pre-crosslinked material to obtain detection data of dielectric property, and
comparing the reference data of dielectric property and the detection data of dielectric property to verify deterioration of the expired pre-crosslinked material.

3. The method for detecting storage life of pre-crosslinked material for high-voltage alternating-current cable insulation according to claim 1, wherein measuring a cross-linking degree of the reference cross-linked polyethylene to obtain reference data of cross-linking degree specifically comprises:
measuring the cross-linking degree of the crosslinked polyethylene by differential scanning calorimetry (DSC) test and thermal extension test to obtain the reference data of cross-linking degree.

4. The method for detecting storage life of pre-crosslinked material for high-voltage alternating-current cable insulation according to claim 1, wherein measuring a cross-linking degree of the crosslinked polyethylene prepared from the pre-crosslinked material that undergoes accelerated aging to obtain detection data of cross-linking degree specifically comprises:
measuring the cross-linking degree of the crosslinked polyethylene prepared from the pre-crosslinked material that undergoes accelerated aging by differential scanning calorimetry (DSC) test and thermal extension test to obtain the detection data of cross-linking degree.

5. The method for detecting storage life of pre-crosslinked material for high-voltage alternating-current cable insulation according to claim 1, wherein the mechanical properties comprise tensile strength, elastic modulus and elongation at break.

6. The method for detecting storage life of pre-crosslinked material for high-voltage alternating-current cable insulation according to claim 2, wherein the dielectric properties comprise direct-current conductivity, alternating-current breakdown field strength, relative dielectric constant and dielectric loss tangent.

## Patentansprüche

1. Verfahren zur Erkennung der Lagerfähigkeit von vorvernetztem Material für Hochspannungswechselstromkabelisolierung, umfassend:
Hernehmen eines vorvernetzten Materials und Unterwerfen des vorvernetzten Materials einem Verpressen, um ein vernetztes Referenzpolyethylen zu erhalten, wobei das vorvernetzte Material ein ungealtertes neues vorvernetztes Material ist,
Messen eines Vernetzungsgrads des vernetzten Referenzpolyethylens, um Referenzdaten des Vernetzungsgrads zu erhalten,
Prüfen mechanischer Eigenschaften des vernetzten Referenzpolyethylens, um Referenzdaten der mechanischen Eigenschaft zu erhalten,
Erhitzen des vorvernetzten Materials bei einer voreingestellten Temperatur für eine voreingestellte Dauer, um ein vorvernetztes Material zu erhalten, das eine beschleunigte Alterung durchläuft,
Unterwerfen des vorvernetzten Materials, das eine beschleunigte Alterung durchläuft, einem Verpressen, um ein vernetztes Polyethylen zu erhalten,
Messen eines Vernetzungsgrads des aus dem vorvernetzten Material, das eine beschleunigte Alterung durchläuft, hergestellten vernetzten Polyethylens, um Erkennungsdaten des Vernetzungsgrads zu erhalten,
Prüfen mechanischer Eigenschaften des aus dem vorvernetzten Material, das eine beschleunigte Alterung durchläuft, hergestellten vernetzten Polyethylens, um Erkennungsdaten der mechanischen Eigenschaft zu erhalten,
Vergleichen der Referenzdaten des Vernetzungsgrads mit den Erkennungsdaten des Vernetzungsgrads, um ein erstes Vergleichsergebnis zu erhalten,
Vergleichen der Referenzdaten der mechanischen Eigenschaft mit den Erkennungsdaten der mechanischen Eigenschaft, um ein zweites Vergleichsergebnis zu erhalten,
Verlängern einer voreingestellten Heizdauer nach einer voreingestellten Schrittweite in dem Fall, dass das erste Vergleichsergebnis innerhalb eines ersten zulässigen Fehlerbereichs liegt und das zweite Vergleichsergebnis innerhalb eines zweiten zulässigen Fehlerbereichs liegt, erneutes Prüfen des vernetzten Polyethylens, um die Erkennungsdaten des Vernetzungsgrads und die Erkennungsdaten der mechanischen Eigenschaft zu erhalten, und
Vergleichen mit den Referenzdaten des Vernetzungsgrads und den Referenzdaten der mechanischen Eigenschaft, bis das erste Vergleichsergebnis nicht innerhalb des ersten zulässigen Fehlerbereichs liegt oder das zweite Vergleichsergebnis nicht innerhalb des zweiten zulässigen Fehlerbereichs liegt,
Aufzeichnen der Heizdauer in dem Fall, dass das erste Vergleichsergebnis nicht innerhalb des ersten zulässigen Fehlerbereichs liegt oder das zweite Vergleichsergebnis nicht innerhalb des zweiten zulässigen Fehlerbereichs liegt, wobei das vorvernetzte Material, das eine beschleunigte Alterung durchläuft, ein abgelaufenes vorvernetztes Material ist,
Erhalten einer Formel zur Berechnung einer Zersetzungsrate eines Vernetzungsmittels in dem vorvernetzten Material: $X = 1 - {e}^{- \frac{t ⋅ ln 2}{τ}}$
wobei τ eine Halbwertszeit des Vernetzungsmittels bei der Temperatur T ist und t eine Zeitdauer ist, während der das Vernetzungsmittel bei der Temperatur T gelagert wird, und
Umwandeln einer Zeitdauer nach Subtrahieren der Schrittweite von der Heizdauer des abgelaufenen vorvernetzten Materials in eine Zeitdauer bei Normaltemperatur nach einer Formel zur Berechnung der Halbwertszeit des Vernetzungsmittels als Funktion der Temperatur, um dadurch die Lagerfähigkeit des vorvernetzten Materials zu erhalten.

2. Verfahren zur Erkennung der Lagerfähigkeit von vorvernetztem Material für Hochspannungswechselstromkabelisolierung nach Anspruch 1, weiter umfassend:
Messen dielektrischer Eigenschaften des vernetzten Referenzpolyethylens, um Referenzdaten der dielektrischen Eigenschaft zu erhalten,
Messen dielektrischer Eigenschaften des aus dem abgelaufenen vorvernetzten Material hergestellten vernetzten Polyethylens, um Erkennungsdaten der dielektrischen Eigenschaft zu erhalten, und
Vergleichen der Referenzdaten der dielektrischen Eigenschaft und der Erkennungsdaten der dielektrischen Eigenschaft, um eine Verschlechterung des abgelaufenen vorvernetzten Materials zu verifizieren.

3. Verfahren zur Erkennung der Lagerfähigkeit von vorvernetztem Material für Hochspannungswechselstromkabelisolierung nach Anspruch 1, wobei das Messen eines Vernetzungsgrads des vernetzten Referenzpolyethylens, um Referenzdaten des Vernetzungsgrads zu erhalten, konkret umfasst:
Messen des Vernetzungsgrads des vernetzten Polyethylens durch dynamische Differenzkalorimetrie-(DSC)-Prüfung und Wärmedehnungsprüfung, um die Referenzdaten des Vernetzungsgrads zu erhalten.

4. Verfahren zur Erkennung der Lagerfähigkeit von vorvernetztem Material für Hochspannungswechselstromkabelisolierung nach Anspruch 1, wobei das Messen eines Vernetzungsgrads des aus dem vorvernetzten Material, das eine beschleunigte Alterung durchläuft, hergestellten vernetzten Polyethylens, um Erkennungsdaten des Vernetzungsgrads zu erhalten, konkret umfasst:
Messen des Vernetzungsgrads des aus dem vorvernetzten Material, das eine beschleunigte Alterung durchläuft, hergestellten vernetzten Polyethylens durch dynamische Differenzkalorimetrie-(DSC)-Prüfung und Wärmedehnungsprüfung, um die Erkennungsdaten des Vernetzungsgrads zu erhalten.

5. Verfahren zur Erkennung der Lagerfähigkeit von vorvernetztem Material für Hochspannungswechselstromkabelisolierung nach Anspruch 1, wobei die mechanischen Eigenschaften Zugfestigkeit, Elastizitätsmodul und Bruchdehnung umfassen.

6. Verfahren zur Erkennung der Lagerfähigkeit von vorvernetztem Material für Hochspannungswechselstromkabelisolierung nach Anspruch 2, wobei die dielektrischen Eigenschaften Gleichstromleitfähigkeit, Wechselstrom-Durchschlagfeldstärke, relative Dielektrizitätskonstante und dielektrischen Verlustfaktor umfassen.

## Revendications

1. Procédé de détection de la durée de conservation d'un matériau pré-réticulé d'isolation de câble à courant alternatif à haute tension, comprenant :
le fait de prendre un matériau pré-réticulé, et de soumettre le matériau pré-réticulé à une mise en plaque, pour obtenir un polyéthylène réticulé de référence, le matériau pré-réticulé étant un nouveau matériau pré-réticulé non vieilli,
le fait de mesurer un degré de réticulation du polyéthylène réticulé de référence, pour obtenir des données de référence de degré de réticulation,
le fait de tester des propriétés mécaniques du polyéthylène réticulé de référence, pour obtenir des données de référence de propriété mécanique,
le fait de chauffer le matériau pré-réticulé à une température prédéfinie pendant une durée prédéfinie, pour obtenir un matériau pré-réticulé qui subit un vieillissement accéléré,
le fait de soumettre le matériau pré-réticulé qui subit un vieillissement accéléré à une mise en plaque, pour obtenir un polyéthylène réticulé,
le fait de mesurer un degré de réticulation du polyéthylène réticulé préparé à partir du matériau pré-réticulé qui subit un vieillissement accéléré, pour obtenir des données de détection de degré de réticulation,
le fait de tester des propriétés mécaniques du polyéthylène réticulé préparé à partir du matériau pré-réticulé qui subit un vieillissement accéléré, pour obtenir des données de détection de propriété mécanique,
le fait de comparer les données de référence de degré de réticulation avec les données de détection de degré de réticulation, pour obtenir un premier résultat de comparaison,
le fait de comparer les données de référence de propriété mécanique avec les données de détection de propriété mécanique, pour obtenir un deuxième résultat de comparaison,
le fait de prolonger, d'une durée d'étape prédéfinie, une durée de chauffage prédéfinie dans le cas où le premier résultat de comparaison est situé à l'intérieur d'une première plage d'erreur admissible et où le deuxième résultat de comparaison est situé à l'intérieur d'une deuxième plage d'erreur admissible, de tester à nouveau le polyéthylène réticulé pour obtenir les données de détection de degré de réticulation et les données de détection de propriété mécanique, et
le fait de comparer avec les données de référence de degré de réticulation et les données de référence de propriété mécanique jusqu'à ce que le premier résultat de comparaison ne soit pas situé à l'intérieur de la première plage d'erreur admissible, ou que le deuxième résultat de comparaison ne soit pas situé à l'intérieur de la deuxième plage d'erreur admissible,
le fait d'enregistrer la durée de chauffage dans le cas où le premier résultat de comparaison n'est pas situé à l'intérieur de la première plage d'erreur admissible ou dans le cas où le deuxième résultat de comparaison n'est pas situé à l'intérieur de la deuxième plage d'erreur admissible, le matériau pré-réticulé qui subit un vieillissement accéléré étant un matériau pré-réticulé périmé,
le fait d'obtenir une formule de calcul d'un taux de décomposition d'un agent de réticulation dans le matériau pré-réticulé : $X = 1 - {e}^{- \frac{t ⋅ ln 2}{τ}}$
dans laquelle τ est une demi-vie de l'agent de réticulation à la température T, et t est une durée pendant laquelle l'agent de réticulation est stocké à la température T, et
le fait de convertir une durée après une soustraction consistant à soustraire la durée de l'étape de la durée de chauffage du matériau pré-réticulé périmé, en une durée à une température normale selon une formule de calcul de la demi-vie de l'agent de réticulation en fonction de la température, de manière à obtenir la durée de conservation du matériau pré-réticulé.

2. Procédé de détection de la durée de conservation d'un matériau pré-réticulé d'isolation de câble à courant alternatif à haute tension selon la revendication 1, comprenant en outre : le fait de mesurer des propriétés diélectriques du polyéthylène réticulé de référence, pour obtenir des données de référence de propriété diélectrique, le fait de mesurer des propriétés diélectriques du polyéthylène réticulé préparé à partir du matériau pré-réticulé périmé, pour obtenir des données de détection de propriété diélectrique, et de comparer les données de référence de propriété diélectrique et les données de détection de propriété diélectrique, pour vérifier la détérioration du matériau pré-réticulé périmé.

3. Procédé de détection de la durée de conservation d'un matériau pré-réticulé d'isolation de câble à courant alternatif à haute tension selon la revendication 1, dans lequel la mesure d'un degré de réticulation du polyéthylène réticulé de référence pour obtenir des données de référence de degré de réticulation comprend spécifiquement : le fait de mesurer le degré de réticulation du polyéthylène réticulé par un essai de calorimétrie différentielle à balayage (DSC) et un essai d'allongement thermique, pour obtenir les données de référence de degré de réticulation.

4. Procédé de détection de la durée de conservation d'un matériau pré-réticulé d'isolation de câble à courant alternatif à haute tension selon la revendication 1, dans lequel la mesure d'un degré de réticulation du polyéthylène réticulé préparé à partir du matériau pré-réticulé qui subit un vieillissement accéléré, pour obtenir des données de détection de degré de réticulation, comprend spécifiquement :
le fait de mesurer le degré de réticulation du polyéthylène réticulé préparé à partir du matériau pré-réticulé qui subit un vieillissement accéléré par un essai de calorimétrie différentielle à balayage (DSC) et un essai d'allongement thermique, pour obtenir les données de détection de degré de réticulation.

5. Procédé de détection de la durée de conservation d'un matériau pré-réticulé d'isolation de câble à courant alternatif à haute tension selon la revendication 1, dans lequel les propriétés mécaniques comprennent la résistance à la traction, le module d'élasticité et l'allongement à la rupture.

6. Procédé de détection de la durée de conservation d'un matériau pré-réticulé d'isolation de câble à courant alternatif à haute tension selon la revendication 2, dans lequel les propriétés diélectriques comprennent la conductivité en courant continu, l'intensité du champ de claquage en courant alternatif, la constante diélectrique relative et la tangente de perte diélectrique.
